# EUROPÄISCHE PATENTSCHRIFT

(11) **EP 4 019 941 B1**
(45) Veröffentlichungstag und Bekanntmachung des Hinweises auf die Patenterteilung: **11.02.2026**
(21) Anmeldenummer: 20216331.7
(22) Anmeldetag: 22.12.2020
(51) Int. Cl.: G01N 21/17, G01N 21/75, G01N 29/02, G01N 29/24, G01N 29/42, G01N 33/00

(54) **VERFAHREN UND VORRICHTUNG ZUM BESTIMMEN EINER EIGENSCHAFT EINES FLUIDS**
METHOD AND DEVICE FOR DETERMINING A CHARACTERISTIC OF A FLUID
PROCÉDÉ ET DISPOSITIF POUR DÉTERMINER UNE PROPRIÉTÉ D'UN FLUIDE

(43) Veröffentlichungstag der Anmeldung: 29.06.2022
(73) Patentinhaber: Fraunhofer-Gesellschaft zur Förderung der angewandten Forschung e.V., 80686 München (DE)
(72) Erfinder: WEBER, Christian, 79110 Freiburg (DE); KAPP, Johannes, 79110 Freiburg (DE); SCHMITT, Katrin, 79110 Freiburg (DE)
(74) Vertreter: WSL Patentanwälte Partnerschaft mbB

(56) Entgegenhaltungen:
- WO-A1-01/57498
- CN-A- 109 507 115
- US-A1- 2005 160 791
- S.L FIREBAUGH ET AL: "Miniaturization and integration of photoacoustic detection with a microfabricated chemical reactor system", JOURNAL OF MICROELECTROMECHANICAL SYSTEMS, June 2001 (2001-06-01), New York, pages 232 - 237, XP055314336, Retrieved from the Internet <URL:http://ieeexplore.ieee.org/ielx5/84/20030/00925756.pdf?tp=&arnumber=925756&isnumber=20030> [retrieved on 20161026], DOI: 10.1109/84.925756
- MURAKAMI ET AL: "In situ observation of photocatalytic reaction by photoacoustic spectroscopy: Detection of heat of exothermic photocatalytic reaction", CHEMICAL PHYSICS LETTERS, ELSEVIER BV, NL, vol. 451, no. 4-6, 15 December 2007 (2007-12-15), pages 316 - 320, XP022418843, ISSN: 0009-2614, DOI: 10.1016/J.CPLETT.2007.12.014
- KIM J-I ET AL: "LASER-INDUCED PHOTOACOUSTIC SPECTROSCOPY FOR THE SPECIATION OF TRANSURANIC ELEMENTS IN NATURAL AQUATIC SYSTEMS", TOPICS IN CURRENT CHEMISTRY; SPRINGER, vol. 157, 1990, BERLIN, DE, pages 130 - 179, XP000671339, ISSN: 0340-1022
- BELLAICHE-SHARPE P ET AL: "REAL-TIME PHOTOACOUSTIC SIGNAL ANALYSIS IN A GAS-PHASE SPECTROPHONE: A FEASIBILITY STUDY", APPLIED SPECTROSCOPY, THE SOCIETY FOR APPLIED SPECTROSCOPY. BALTIMORE, US, vol. 50, no. 11, 1996, pages 1366 - 1372, XP000642383, ISSN: 0003-7028, DOI: 10.1366/0003702963904647

## Beschreibung

Die vorliegende Erfindung betrifft ein Verfahren zum Bestimmen einer Eigenschaft eines Fluids mit den Schritten: A) Erzeugen einer mit einer Modulationsfrequenz modulierten elektromagnetischen Anregungsstrahlung, wobei eine Trägerfrequenz der Anregungsstrahlung derart gewählt wird, dass ein erstes Fluid die Anregungsstrahlung absorbiert, B) Beleuchten des ersten Fluids mit der Anregungsstrahlung, C) Erfassen einer durch eine Absorption der Anregungsstrahlung in dem ersten Fluid erzeugten Schallwelle mit einer Schallmesseinrichtung.

Die vorliegende Erfindung betrifft darüber hinaus eine Vorrichtung zum Bestimmen einer Eigenschaft eines Fluids mit einem ersten Fluid, mit einer Quelle, die derart eingerichtet ist, dass die Quelle in einem Betrieb der Vorrichtung mit einer Modulationsfrequenz modulierte elektromagnetische Anregungsstrahlung erzeugt, wobei eine Trägerfrequenz der Anregungsstrahlung derart gewählt ist, dass ein erstes Fluid die Anregungsstrahlung absorbiert, wobei die Quelle derart angeordnet ist, dass in dem Betrieb der Vorrichtung das erste Fluid mit der Anregungsstrahlung beleuchtbar ist, und mit einer Schallmesseinrichtung, wobei die Schallmesseinrichtung derart eingerichtet und angeordnet ist, dass in dem Betrieb der Vorrichtung eine durch eine Absorption der Anregungsstrahlung in dem ersten Fluid erzeugte Schallwelle mit der Schallmesseinrichtung erfassbar ist.

Aus ganz unterschiedlichen Gründen wächst die Nachfrage nach Analyseverfahren und Vorrichtungen zum Bestimmen einer Eigenschaft eines Fluids, insbesondere eines Zielgases innerhalb eines Gasgemisches. Zum Bestimmen einer Eigenschaft eines solchen Fluids können neben den absorptionsspektrometrischen Verfahren auch photoakustische Verfahren und Sensoren eingesetzt werden.

Bei einem photoakustischen Verfahren wird das Fluid, insbesondere ein das Zielgas enthaltenes Gasgemisch, mit einer elektromagnetischen Anregungsstrahlung bestrahlt, wobei die Anregungsstrahlung eine Trägerfrequenz, d.h. eine Frequenz ihres elektromagnetischen Wechselfeldes, aufweist, die gleich einer Absorptionsfrequenz des Fluids ist. Dadurch absorbiert das Fluid die Anregungsstrahlung. Es kommt aufgrund der Absorption zu einer Erwärmung und somit zu einer thermischen Ausdehnung des Fluids.

Damit aus der Bestrahlung des Fluids mit der Anregungsstrahlung Rückschlüsse auf eine Eigenschaft des Fluids gezogen werden können, wird die Anregungsstrahlung derart moduliert, dass die thermische Ausdehnung des Fluids periodisch erfolgt und somit eine Schallwelle innerhalb des Fluids generiert wird. Durch eine Messung zumindest der Amplitude oder der Phase dieser Schallwelle mit einer geeigneten Schallmesseinrichtung kann auf die Eigenschaft des Fluids zurückgeschlossen werden.

Photoakustische Verfahren und die zugehörigen Vorrichtungen haben das Potenzial, insbesondere preisgünstige Alternativen zu optischen Verfahren der Spektroskopie zu bilden.

Die Möglichkeit zur Adressierung von Fluiden mit einem solchen photoakustischen Verfahren hängt von der Zugänglichkeit von Quellen für die geeignete Anregungsstrahlung ab. Die Frequenz des elektromagnetischen Wechselfeldes muss so gewählt sein, dass das Fluid bei dieser Frequenz absorbiert. Dies stellt sich bisweilen als entweder teuer oder gar unmöglich dar. Daher ist es eine Aufgabe der vorliegenden Erfindung, ein Verfahren und eine Vorrichtung zum Bestimmen einer Eigenschaft eines Fluids bereitzustellen, die es ermöglichen, auch ein Fluid zu adressieren, für das elektromagnetische Anregungsstrahlung bei der Absorptionsfrequenz mit herkömmlichen Quellen nicht oder nur sehr aufwendig generierbar ist.

Darüber hinaus kommt es bei photoakustischen Verfahren immer wieder zu nicht eindeutigen Ergebnissen in dem Sinne, dass nicht zu unterscheiden ist, ob die erfasste Schallwelle ursächlich auf eine Absorption der Anregungsstrahlung in dem zu untersuchenden Fluid oder auf eine andere Ursache zurückgeht.

Die WO 01/57498 A1 beschreibt die Durchführung einer chemischen oder physikalischen oder chemischen und physikalischen Umsetzung eines Edukts, wobei ein Abstrom umfassend mindestens ein Umsetzungsprodukt erhalten wird. Die Analyse des bei der Umsetzung erhaltenen Abstroms wird dabei durch Aufnahme und Auswertung mindestens eines photoakustischen Signals durchgeführt. Ein dabei verwendbares photoakustisches Detektionssystem enthält folgende wesentliche Merkmale:
1. Molekülschwingungen, Rotationsbewegungen, elektronische Anregungszustände, chemische Reaktionen oder Kombinationen aus diesen Prozessen chemischer Verbindungen, die sich im Umsetzungsprodukt und/oder dem Edukt befinden, anregende Lichtquelle;
2. mindestens eine Messeinrichtung zur Aufnahme der photoakustischen Signale, z. B. Druckgradientenwandler wie Mikrofone, Druckwandler wie Barometer, oder Kombinationen aus diesen, wie z. B. Ultraschallwandler;
3. mindestens eine Datenaufnahme- und Auswertungseinheit zur Auswertung der erzeugten photoakustischen Signale, sowie
4. gegebenenfalls eine Auffangeinrichtung, vorzugsweise eine Messküvette,
in der der Abstrom gasförmig, flüssig oder fest enthalten ist, wobei jedoch im einfachsten Fall auch im freien Gasraum gearbeitet werden kann.

Die wissenschaftliche Veröffentlichung von S.L. Firebaugh et al. "Miniaturization and integration of photoacoustic detection with a microfabricated chemical reactor system", Journal of Microelectromechanical Systems, Juni 2001, Seiten 232-237, New York offenbart die Miniaturisierung der photoakustischen Detektion und verwendet insbesondere 3,4-Mikrometer-Licht, um Propan in einem Kohlendioxid-Hintergrund zu erkennen. Dabei wurde eine Miniatur-Photoakustikzelle in den Montageblock eines mikrogefertigten chemischen Reaktors eingearbeitet, was die Integration eines photoakustischen Detektors in ein miniaturisiertes System demonstriert. Die Zelle verwendete ein Hörgerätemikrofon und eine Infrarotdiode, die bei der ersten akustischen Resonanz der Zelle moduliert wurde. Als sich die Gaszusammensetzung der Zelle von Kohlendioxid zu Propan änderte, wurde beobachtet, dass sich der Resonanzpeak verschob und vergrößerte, wie es von der Theorie erwartet worden war.

Die wissenschaftliche Veröffentlichung von: N. Murakami et al. "In situ observation of photocatalytic reaction by photoacoustic spectroscopy: Detection of heat of exothermic photocatalytic reaction", Cchemical Physics Letters, Elsevier BV, NL, Bd. 451, Nr. 4-6, 15. Dezember 2007, Seiten 316-320 offenbart die Anwendung von modulationsfrequenzabhängiger photoakustischer Spektroskopie auf goldbeschichtete Titan(IV)-Oxid-Pulver in Gegenwart von adsorbiertem Methanol, um das photoakustische Signal aufgrund von Phänomenen, die während photokatalytischer Reaktionen auftreten, z. B. die Freisetzung von Reaktionswärme, zu erfassen. Die Analyse der Spitzenfrequenz der Helmholtz-Resonanz lieferte qualitative Informationen über die durch photokatalytische Reaktionen (Oxidation oder Dehydrierung von Methanol) hervorgerufenen Veränderungen der Gaszusammensetzung, die von der An- oder Abwesenheit von Sauerstoff abhingen. Das photoakustische Signal, das der Wärme der photokatalytischen Reaktion zuzuschreiben ist, wurde beobachtet, indem die Wirkung der Änderungen in der Gaszusammensetzung mit zwei Arten von moduliertem Licht als Anregung und Nichtanregung für die photokatalytische Reaktion aufgehoben wurde.

Daher ist es auch eine Aufgabe der vorliegenden Erfindung, ein Verfahren und eine Vorrichtung zum Bestimmen einer Eigenschaft eines Fluids bereitzustellen, die eine erhöhte Eindeutigkeit der Aussagefähigkeit bei der Erfassung der Schallwelle aufweisen.

Die vorliegende Erfindung ist in den Ansprüchen 1 und 6 definiert.

Zumindest eine der zuvor genannten Aufgaben wird durch ein Verfahren gemäß dem unabhängigen Anspruch 1 gelöst. Dabei weist das Verfahren der eingangs genannten Art weiterhin die Schritte auf:
D) zeitlich zumindest vor oder während des Beleuchtens des ersten Fluids in Schritt B) Initiieren einer chemischen Reaktion,
E) Umwandeln des ersten Fluids als Reaktionsedukt in ein zweites Fluid als Reaktionsprodukt in der chemischen Reaktion und
F) Bestimmen eines Maßes für eine Eigenschaft des ersten Fluids aus zumindest einer Amplitude oder einer Phase der durch die Absorption der Anregungsstrahlung durch das erste Fluid erzeugten Schallwelle.

Darüber hinaus werden zeitlich vor dem Initiieren der chemischen Reaktion in Schritt D) die Schritte ausgeführt
G) Beleuchten des ersten Fluids mit der Anregungsstrahlung (3),
H) Erfassen einer durch die Absorption der Anregungsstrahlung (3) in dem ersten Fluid erzeugten Schallwelle mit der Schallmesseinrichtung (5, 12),
I) Bestimmen eines Vergleichsmaßes für die Eigenschaft des ersten Fluids aus zumindest der Amplitude oder der Phase der durch die Absorption der Anregungsstrahlung (3) durch das erste Fluid erzeugten Schallwelle und
   weiterhin umfasst das Verfahren den Schritt
J) Bestimmen eines differenziellen Maßes für die Eigenschaft des ersten Fluids aus dem Maß für die Eigenschaft des ersten Fluids aus Schritt F) und dem Vergleichsmaß für die Eigenschaft des ersten Fluids aus Schritt I).

Der vorliegenden Erfindung liegt die Idee zu Grunde, eine chemische Umwandlungsreaktion, bei welcher das erste Fluid entweder als Reaktionsedukt beteiligt ist, zur Erfassung der Eigenschaft des ersten Fluids zu nutzen.

Im Sinne der vorliegenden Anmeldung bezeichnet das erste Fluid dasjenige Fluid, welches die Anregungsstrahlung absorbiert und durch dessen Absorption die Schallwelle erzeugt wird.

Im Sinne der vorliegenden Anmeldung ist das Fluid, insbesondere das erste Fluid oder das zweite Fluid, eine Flüssigkeit oder ein Gas, insbesondere ein Gas in einem Gasgemisch.

Erfindungsgemäß ist das erste Fluid als Reaktionsedukt an der chemischen Umwandlungsreaktion beteiligt. Gleichzeitig ist das erste Fluid dasjenige, dessen Eigenschaft es zu bestimmen gilt. Durch die chemische Reaktion lässt sich die Konzentration des ersten Fluids gezielt ändern, sodass erkennbar ist, ob die erfasste Schallwelle ursächlich auf die Absorption der Anregungsstrahlung in dem ersten Fluid zurückgeht oder nicht.

Erfindungsgemäß ist das erste Fluid das Ziel- oder Probenfluid, dessen Eigenschaft bestimmt wird.

In einer Ausführungsform der Erfindung ist die Eigenschaft des ersten Fluids ein Vorhandensein oder eine Konzentration des ersten Fluids.

Im Sinne der vorliegenden Anmeldung bezeichnet die Trägerfrequenz der Anregungsstrahlung die Frequenz des elektromagnetischen Wechselfeldes der Anregungsstrahlung.

In einer Ausführungsform der Erfindung wird die Intensität der Anregungsstrahlung mit der Modulationsfrequenz moduliert. In einer weiteren Ausführungsform wird die Trägerfrequenz der Anregungsstrahlung mit der Modulationsfrequenz moduliert. Entscheidend ist, dass durch die aufgeprägte Modulation die Anregung in dem ersten Fluid moduliert bzw. ein- und ausgeschaltet wird. Durch die periodische Modulation der Anregungsstrahlung wird eine Schallwelle erzeugt, deren Schallfrequenz gleich der Modulationsfrequenz der Anregungsstrahlung ist.

Erfindungsgemäß umfasst das Verfahren in einem weiteren Schritt D) ein Initiieren der chemischen Reaktion. Das gezielte Initiieren der chemischen Reaktion, d.h. der Umwandlung des ersten Fluids in das zweite Fluid, ermöglicht es, durch die Steuerung der chemischen Reaktion zielgenau das Maß für die Eigenschaft des ersten Fluids zu bestimmen. Das Initiieren der chemischen Reaktion ermöglicht die Unterscheidung zweier Messungen, einmal mit und einmal ohne chemische Reaktion. Auf diese Weise können beispielsweise Hintergrundeffekte erkannt und herausgerechnet werden.

Mit anderen Worten ausgedrückt, ist die chemische Reaktion steuerbar in dem Sinne, dass sie einschaltbar ist. In einer Ausführungsform der Erfindung ist die chemische Reaktion mit einer Reaktionsmodulationsfrequenz modulierbar, d.h. periodisch ein- und ausschaltbar.

In einer Ausführungsform der Erfindung erfolgt das Initiieren der chemischen Reaktion in Schritt D) durch zumindest einen der folgenden Schritte:
- Zuführen von thermischer Energie in das Reaktionsedukt,
- Bestrahlen des Reaktionsedukts mit elektromagnetischer Aktivierungsstrahlung,
- Einbringen eines Katalysators in das Reaktionsedukt,
- Aktivieren eines Katalysators in dem Reaktionsedukt oder
- chemisches Aktivieren des Reaktionsedukts.

Eine Möglichkeit zum Zuführen von thermischer Energie ist das Aufheizen mit einem elektrischen Heizelement, einer Zündeinrichtung oder elektromagnetischer Strahlung, welche durch Absorption Wärme erzeugt.

Ein weiteres Beispiel für das Zuführen thermischer Energie ist eine Gasentladung zwischen zwei Elektroden.

In einer Ausführungsform erfolgt das Umwandeln mit der chemischen Reaktion durch Elektrolyse. Eine Elektrolyse lässt sich durch Anlegen einer Spannung an zwei mit dem Elektrolyten in Kontakt stehenden Elektroden initiieren. In einer solchen Ausführungsform ist vorzugsweise zumindest das erste oder das zweite Fluid eine wässrige Lösung oder ein Plasma.

Auch ist es möglich, über die thermische Initiierung hinaus durch das Bestrahlen des Reaktionsedukts mit elektromagnetischer Aktivierungsstrahlung die chemische Reaktion zu initiieren, indem das Reaktionsedukt in einen angeregten Zustand versetzt wird.

Im Falle einer Bestrahlung des Reaktionsedukts mit elektromagnetischer Aktivierungsstrahlung kann diese Aktivierungsstrahlung die gleiche oder eine andere Trägerfrequenz aufweisen wie die Anregungsstrahlung.

Wenn in einer Ausführungsform der Erfindung das Initiieren der chemischen Reaktion in Schritt D) durch Bestrahlen des Reaktionsedukts mit der elektromagnetischen Anregungsstrahlung erfolgt, so weist die elektromagnetische Anregungsstrahlung eine zwischen einem ersten Wert zeitlich vor dem Schritt D) und einem zweiten Wert zeitlich während einer Ausführung des Schritts D) veränderbare Modulationstiefe auf. In einer Ausführungsform ist der zweite Wert der Modulationstiefe kleiner als der erste Wert. Mit anderen Worten ausgedrückt, wird während einer Ausführung des Schritts D) die Anregungsstrahlung mit einem nicht modulierten DC-Anteil versehen. Dieser DC-Anteil initiiert ausschließlich die chemische Reaktion, ohne aber die durch die Absorption generierte Schallwelle zu beeinflussen.

Unter der Modulationstiefe im Sinne der vorliegenden Anmeldung wird das Verhältnis zwischen einem maximalen Wert und einem minimalen Wert der Intensität der Anregungsstrahlung verstanden. Eine Modulation, bei der der minimale Wert der Intensität 0 ist, weist eine maximale Modulationstiefe von 1 auf. In einer Ausführungsform wird dabei die Amplitude der Intensitätsmodulation der Anregungsstrahlung in dem Maße vergrößert, wie die Modulationstiefe verringert wird. Dann ist der absolute Hub zwischen dem minimalen Wert und dem maximalen Wert der Intensität konstant, während durch das Hinzufügen des DC-Anteils die Modulationstiefe verringert wird.

Ein chemisches Aktivieren des Reaktionsedukts kann beispielsweise durch Einspritzen eines reaktiven Fluids erfolgen.

Darüber hinaus gibt es die Möglichkeit, die chemische Reaktion katalytisch zu initiieren. Dazu kann entweder ein Katalysator in das Reaktionsedukt eingebracht werden oder aber ein solcher Katalysator, beispielsweise thermisch, aktiviert werden.

Erfindungsgemäß werden zeitlich vor dem Initiieren der chemischen Reaktion in Schritt D) die Schritte ausgeführt:
G) Beleuchten des ersten Fluids mit der Anregungsstrahlung,
H) Erfassen einer durch die Absorption der Anregungsstrahlung in dem ersten Fluid erzeugten Schallwelle mit der Schallmesseinrichtung,
I) Bestimmen eines Vergleichsmaßes für die Eigenschaft des ersten Fluids aus zumindest der Amplitude oder der Phase der durch die Absorption der Anregungsstrahlung durch das erste Fluid erzeugten Schallwelle und
   das Verfahren weist weiterhin den Schritt auf
J) Bestimmen eines differenziellen Maßes für die Eigenschaft des ersten Fluids aus dem Maß für die Eigenschaft des ersten Fluids aus Schritt G) und dem Vergleichsmaß für die Eigenschaft des ersten Fluids aus Schritt K).

In dieser Ausführungsform wird die Eigenschaft des ersten Fluids zweimal bestimmt. Ein Vergleichsmaß für die Eigenschaft wird vor dem Initiieren der chemischen Reaktion bestimmt und das Maß für die Eigenschaft wird nach dem Initiieren der chemischen Reaktion ein zweites Mal bestimmt. Aus dem Vergleichsmaß und dem Maß lässt sich ein differenzielles Maß bestimmen und es ist erkennbar, welcher Anteil der erfassten Schallwelle tatsächlich auf die Absorption durch das erste Fluid zurückgeht.

Nachfolgend wird ein Beispiel für das erfindungsgemäße Verfahren beschrieben.

In einer Ausführungsform der Erfindung wird das Verfahren zum Nachweis von Stickstoffdioxid (NO₂) oder zum Erfassen einer Konzentration von Stickstoffdioxid verwendet. Dabei bildet das Stickstoffdioxid das erste Fluid im Sinne der vorliegenden Anmeldung. In Schritt F) wird das Stickstoffdioxid durch eine chemische Reaktion in Stickstoffmonoxid umgewandelt. Das Stickstoffmonoxid bildet das zweite Fluid im Sinne der vorliegenden Erfindung. Durch die chemische Reaktion wird selektiv die Konzentration des Stickstoffdioxids reduziert. Aus einem Vergleich mit und ohne chemische Reaktion kann das Stickstoffdioxid als Ursprung der Schallwelle eindeutig identifiziert werden. In einer Ausführungsform der Erfindung wird die chemische Reaktion durch Zuführen thermischer Energie initiiert. In einer Ausführungsform wird die chemische Reaktion durch Bestrahlung mit elektromagnetischer Aktivierungsstrahlung initiiert. In einer Ausführungsform der Erfindung liegt die Anregungsstrahlung im grünen Spektralbereich, während die Aktivierungsstrahlung im ultravioletten Spektralbereich, vorzugsweise in einem Bereich von 350 nm bis 400 nm, liegt.

Darüber hinaus wird zumindest eine der zuvor genannten Aufgaben auch durch eine Vorrichtung zum Bestimmen einer Eigenschaft eines Fluids gemäß dem beigefügten unabhängigen Vorrichtungsanspruch gelöst. Dazu weist die Vorrichtung der eingangs genannten Art weiterhin eine Aktivierungseinrichtung und eine Steuer- und Auswerteeinrichtung auf,
wobei die Steuer- und Auswerteeinrichtung derart wirksam mit der Schallmesseinrichtung verbunden ist, dass die Steuer- und Auswerteeinrichtung in dem Betrieb der Vorrichtung ein Messsignal von der Schallmesseinrichtung erhält, wobei die Steuer- und Auswerteeinrichtung derart wirksam mit der Aktivierungseinrichtung verbunden ist, dass die Aktivierungseinrichtung in dem Betrieb der Vorrichtung von der Steuer- und Auswerteeinrichtung steuerbar ist, und
die Aktivierungseinrichtung derart eingerichtet und angeordnet ist, dass die Aktivierungseinrichtung in dem Betrieb der Vorrichtung eine chemische Reaktion zum Umwandeln des ersten Fluids als Reaktionsedukt in das zweite Fluid als Reaktionsprodukt initiiert, und
die Steuer- und Auswerteeinrichtung derart eingerichtet ist, dass die Steuer- und Auswerteeinrichtung in dem Betrieb der Vorrichtung aus dem Messsignal der durch die Absorption der Anregungsstrahlung durch das erste Fluid erzeugten Schallwelle ein Maß für eine Eigenschaft des ersten Fluids bestimmt.

Ferner ist die Steuer- und Auswerteeinrichtung derart eingerichtet, dass die Steuer- und Auswerteeinrichtung in dem Betrieb der Vorrichtung zeitlich vor dem Initiieren der chemischen Reaktion aus dem Messsignal der durch die Ab-sorption der Anregungsstrahlung durch das erste Fluid erzeugten Schallwelle ein Vergleichsmaß für die Eigenschaft des ersten Fluids bestimmt, und ein differenzielles Maß für die Eigenschaft des ersten Fluids aus dem Maß für die Eigenschaft des ersten Fluids und dem Vergleichsmaß für die Eigenschaft des ersten Fluids bestimmt.

Soweit im folgenden Aspekte der Erfindung im Hinblick auf die Vorrichtung beschrieben werden, so gelten diese auch für das entsprechende Verfahren zum Bestimmen einer Eigenschaft eines Fluids und umgekehrt. Soweit das Verfahren mit einer Vorrichtung gemäß dieser Erfindung ausgeführt wird, so weist diese Vorrichtung die entsprechenden Einrichtungen zum Ausführen des Verfahrens auf. Insbesondere sind Ausführungsformen der erfindungsgemäßen Vorrichtung zum Ausführen der zuvor beschriebenen Ausführungsformen des Verfahrens geeignet.

In einer Ausführungsform der Erfindung ist die Aktivierungseinrichtung ausgewählt aus
- einer Heizeinrichtung zum Zuführen von thermischer Energie in das Reaktionsedukt,
- einer Quelle für elektromagnetische Aktivierungsstrahlung zum Bestrahlen des Reaktionsedukts,
- einem aktivierbaren Katalysator,
- einer Einrichtung zum Einspritzen einer chemischen Substanz zum Aktivieren des Reaktionsedukts
- oder einer Kombination davon.

In einer Ausführungsform der Erfindung ist der aktivierbare Katalysator ein beheizbares Element mit einem Metall, vorzugsweise ein Element der Platingruppe (Ni, Pd, Pt oder Ds), insbesondere Platin oder Palladium, aufweisenden Oberflächenabschnitt.

In einer Ausführungsform der Erfindung weist die Vorrichtung eine Gasmesszelle auf, wobei die Gasmesszelle derart ausgestaltet ist, dass in der Gasmesszelle das Reaktionsedukt und das Reaktionsprodukt aufnehmbar ist und die chemische Reaktion in der Gasmesszelle ablaufen kann, wobei die Schallmesseinrichtung in der Gasmesszelle angeordnet ist. Es versteht sich, dass in einer solchen Ausführungsform die Gasmesszelle so ausgestaltet sein muss, dass die Anregungsstrahlung entweder in die Gasmesszelle einstrahlbar ist oder direkt in dieser mit der Quelle erzeugbar ist.

In einer Ausführungsform der Erfindung ist die Aktivierungseinrichtung, insbesondere eine Heizeinrichtung oder ein Katalysator, in der Gasmesszelle angeordnet.

In einer Ausführungsform der Erfindung bildet die Gasmesszelle einen akustischen Resonator mit einer Eigenfrequenz, wobei die Eigenfrequenz des Resonators gleich der Modulationsfrequenz der Anregungsstrahlung ist. Dies führt zu einer Überhöhung der Amplitude der von der Schallmesseinrichtung zu erfassenden Schallwelle.

In einer Ausführungsform der Erfindung ist die Schallmesseinrichtung ein Wechseldruckwandler oder ein Wechselfluidflusswandler.

Ein Beispiel für einen Wechseldruckwandler ist ein Mikrofon, wobei es auf die konkrete Bauart des Mikrofons erfindungsgemäß nicht ankommt. Ein Wechseldruckwandler erfasst, vorzugsweise zeitaufgelöst, eine Druckänderung in dem Fluid, mit welchem der Wandler in Kontakt ist.

Ein Wechselfluidflusswandler hingegen erfasst, vorzugsweise zeitaufgelöst, eine Änderung eines Flusses eines Fluides, welches den Wandler umgibt. Ein Beispiel für einen Wechselfluidflusswandler ist eine Messeinrichtung, die auf einer Temperaturänderung eines Drahtes beruht, wobei sich der Draht in der Schallwelle befindet.

Weitere Vorteile, Merkmale und Anwendungsmöglichkeiten der vorliegenden Erfindung werden anhand der folgenden Beschreibung von Ausführungsformen und der dazugehörigen Figuren deutlich. In den Figuren sind gleiche Elemente mit gleichen Bezugszeichen bezeichnet.
- Figur 1: ist eine schematische Querschnittsansicht einer ersten Ausführungsform einer Vorrichtung zum Bestimmen einer Eigenschaft eines Fluids.
- Figur 2: ist eine schematische Querschnittsansicht einer zweiten Ausführungsform einer Vorrichtung zum Bestimmen einer Eigenschaft eines Fluids.
- Figur 3: ist eine schematische Querschnittsansicht einer weiteren Ausführungsform einer Vorrichtung zum Bestimmen einer Eigenschaft eines Fluids.

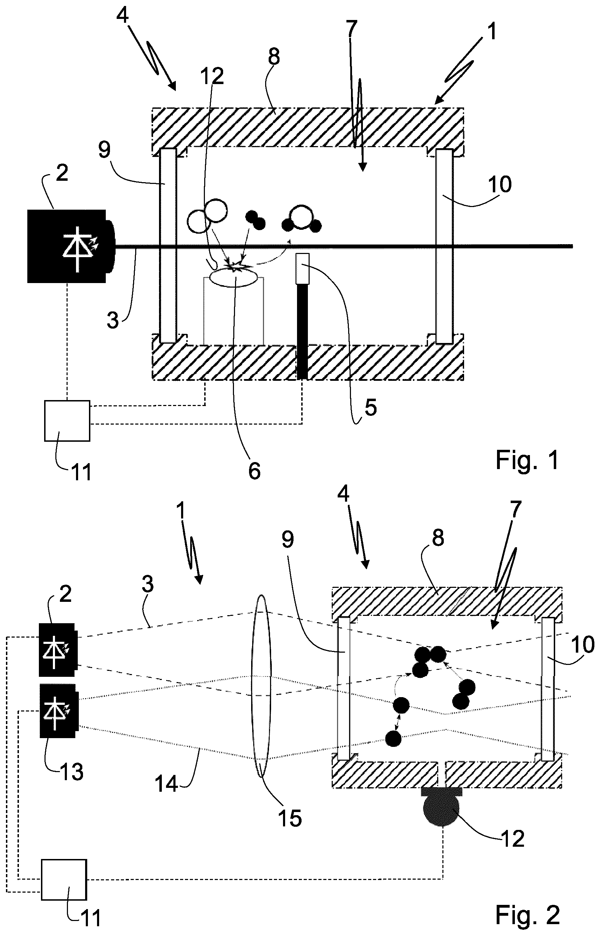

Im Folgenden werden drei Ausführungsformen anhand der Vorrichtung zum Bestimmen einer Eigenschaft eines Fluids aus den Figuren 1 bis 3 im Detail beschrieben. Dabei werden zunächst die Elemente der jeweiligen Vorrichtung mit ihrer individuellen Funktion und dann das Verfahren anhand einer konkreten Verwendung der Vorrichtung beschrieben.

Der photoakustische Sensor 1 aus Figur 1 umfasst eine Laserdiode 2 als Quelle für die elektromagnetische Anregungsstrahlung 3. Der photoakustische Sensor 1 bildet eine Vorrichtung zum Bestimmen eine Eigenschaft eines Fluids im Sinne der vorliegenden Anmeldung. Der Sensor 1 weist weiterhin eine Gasmesszelle 4, einen Wechselgasflusswandler 5 als Schallmesseinrichtung im Sinne der vorliegenden Anmeldung sowie ein katalytisch aktives Heizelement 6 als Aktivierungseinrichtung im Sinne der vorliegenden Anmeldung auf. Der Wechselgasflusswandler 5 beruht auf einem erwärmten Draht, dessen Temperaturänderung aufgrund des vorbeiströmenden Gases erfasst wird.

Die Intensität der elektromagnetischen Anregungsstrahlung 3 ist mit einer Frequenz von 5 kHz moduliert. In der dargestellten Ausführungsform wird diese Intensitätsmodulation der Anregungsstrahlung 3 der Laserdiode 2 durch eine Strommodulation des über die Diode fließenden Stroms aufgeprägt. Absorbiert ein Gas im Innenraum 7 der Zelle die Anregungsstrahlung 3, so wird diese Absorption mittels der Modulationsfrequenz der Anregungsstrahlung 3 an- und ausgeschaltet. Diese Periodizität sorgt dafür, dass die Druckänderung in dem absorbierenden Gas ebenfalls mit der Modulationsfrequenz moduliert ist. Auf diese Weise ist die Frequenz der in dem Innenraum 7 generierten Schallwelle gleich der Modulationsfrequenz der Anregungsstrahlung 3.

Die Gasmesszelle 4 stellt einen im Wesentlichen zylindrischen Innenraum 7 zur Aufnahme des zu messenden Fluids, d. h. in dieser Ausführungsform eines Gases, bereit. Der Innenraum 7 wird von Seitenwänden 8 und zwei Zellenfenstern 9, 10 begrenzt.

Die Zellenfenster dienen dazu, die von der Leuchtdiode 2 erzeugte Anregungsstrahlung 3 durch das Einkoppelfenster 9 in den Innenraum 7 der Gasmesszelle 4 einzukoppeln und durch das Austrittsfenster 10 aus dem Innenraum 7 der Gasmesszelle auszukoppeln. Dabei ist das Material der beiden Fenster 9, 10 so gewählt, dass sie eine minimale Absorption im Bereich der Trägerfrequenz der Anregungsstrahlung 3 zeigen. So kommt es zu möglichst wenig Störungen des Messsignals, da jede Absorption in der Zelle 4 grundsätzlich auch eine Schallwelle nach sich ziehen kann.

Eine durch eine Absorption der Anregungsstrahlung durch ein in dem Innenraum 7 der Zelle 4 befindliches Gas erzeugte Schallwelle ist mit Hilfe des Gasflusssensors 5 erfassbar. Der Gasflusssensor 5 wird von einem beheizbaren Draht gebildet, der sich in der Schallwelle befindet, sodass ein Gasfluss oder Gasstrom den Draht abkühlt. Diese Abkühlung ist im elektrischen Strom durch den Draht messbar. Der Flusssensor 5 ist in einem Flussmaximum dieser stehenden Welle angeordnet, um das messbare Signal zu optimieren.

Der Innenraum 7 der Gasmesszelle 4 ist als Resonator für die entstehende Schallwelle ausgelegt. So bildet sich die Schallwelle in dem Innenraum 7 der Gasmesszelle 4 als stehende Welle aus.

Das katalytische Heizelement 6 umfasst einen elektrisch beheizten Oberflächenabschnitt aus Platin, wobei das Platin als Katalysator wirkt, wenn es eine bestimmte kritische Temperatur übersteigt.

In der dargestellten Ausführungsform der Figur 1 sind die Quelle 2 und das Heizelement 6 mit einer Steuer- und Auswerteeinrichtung 11 verbunden. Ferner ist auch der Flusssensor 5 mit der Steuer- und Auswerteeinrichtung 11 verbunden. Im Betrieb gibt die Steuer- und Auswerteeinrichtung 11 der Diode die Modulationsfrequenz vor und erhält von dem Flusssensor 5 einen Messwert für die Amplitude der in dem Innenraum 7 der Gasmesszelle 4 generierten Schallwelle. Ferner steuert die Steuer- und Auswerteeinrichtung 11 das Heizelement, sodass die Aktivität der katalytischen Oberfläche steuerbar ist.

Die in Figur 1 gezeigte Ausführungsform der vorliegenden Erfindung dient dem Nachweis von Wasserstoff in einem in die Gasmesszelle 4 eingeleiteten Gasgemisch. Der Wasserstoff bildet das zweite Fluid im Sinne der vorliegenden Anmeldung. Da Wasserstoff keine mit kommerziellen Quellen erreichbaren Absorptionsbanden aufweist, erfolgt der Nachweis des Wasserstoffs erfindungsgemäß indirekt.

Die Trägerfrequenz der Anregungsstrahlung 3 ist so gewählt, dass sie von Wasser in der Gasmesszelle absorbiert wird. Die Anregungsstrahlung 3 hat daher eine Trägerwellenlänge von 1,4 µm. Das Wasser bildet das erste Fluid im Sinne der vorliegenden Anmeldung.

Wird nun der Innenraum 7 der Gasmesszelle 4 mit der Anregungsstrahlung 3 beleuchtet, während das Heizelement 6 ausgeschaltet ist, so generiert nur das ohnehin in dem Gasgemisch enthaltene Wasser die Schallwelle mit geringer Amplitude. Schaltet die Steuer- und Auswerteeinrichtung 11 das Heizelement 6 ein, so findet von der katalytischen Oberfläche 12 initiiert eine chemische Reaktion des Wasserstoffs mit dem in dem Gasgemisch enthaltenen Sauerstoff zu Wasser statt. Dieses aus dem Wasserstoff erzeugte Wasser trägt zur Absorption der Anregungsstrahlung 3 bei und erhöht die Amplitude der in dem Innenraum 7 generierten Schallwelle. Zieht man nun das Vergleichsmaß für die Konzentration des Wasserstoffs ohne initiierte Umwandlungsreaktion von dem Maß für die Konzentration des Wasserstoffs bei initiierter Umwandlungsreaktion ab, so erhält man ein eindeutiges differenzielles Maß für die Konzentration des Wasserstoffs in der Gasmesszelle 4.

Der Aufbau des photoakustischen Sensors 1 aus Figur 2 unterscheidet sich insbesondere dadurch von dem Sensor 1 aus Figur 1, dass in dem Innenraum 7 der Zelle 4 kein Heizelement vorgesehen ist. Ferner dient zum Erfassen der Schallwelle in dem Innenraum 7 in dieser Ausführungsform ein Druckwandler, nämlich ein Mikrofon 12. Über die Quelle 2 für die Anregungsstrahlung 3 hinaus weist die Vorrichtung 1 eine Quelle 13 für elektromagnetische Aktivierungsstrahlung 14 auf. Die Quelle 13 für die Aktivierungsstrahlung 14 sowie das Mikrofon 12 sind mit der Steuer- und Auswerteeinrichtung 11 verbunden. Sowohl die Anregungsstrahlung 3 als auch die Aktivierungsstrahlung 14 werden mithilfe einer Linse 15 in den Innenraum 7 der Gasmesszelle 4 fokussiert.

Die in Figur 2 gezeigte Ausführungsform dient in einer ersten Verwendung dem Erfassen einer Konzentration von Sauerstoff in einem Gasgemisch im Innenraum 7 der Gasmesszelle 4. Der Sauerstoff bildet in dieser Ausführungsform das zweite Fluid im Sinne der vorliegenden Anmeldung. Auch die Absorptionsfrequenzen von Sauerstoff liegen außerhalb eines mit kommerziellen Quellen 2 leicht zu adressierenden Bereichs. Daher erfolgt die Erfassung der Konzentration des Sauerstoffs in der Zelle 4 indirekt durch Absorption der Anregungsstrahlung 3 durch Ozon. Das Ozon bildet das erste Fluid im Sinne der vorliegenden Anmeldung.

Dazu wird der Sauerstoff durch eine chemische Reaktion in Sauerstoffradikale aufgespalten, wobei dann die Sauerstoffradikale mit dem Sauerstoff zu dem Ozon reagieren. Die Aufspaltung des Sauerstoffs in Sauerstoffradikale wird durch Bestrahlung des Sauerstoffs mit der Aktivierungsstrahlung 14 initiiert. Auch in dieser Ausführungsform wird wieder eine Messung vor dem Initiieren der chemischen Reaktion durchgeführt, wobei die Quelle 13 für die Aktivierungsstrahlung 14 ausgeschaltet ist. Auf diese Weise kann ein Hintergrund an ohnehin in dem Gasgemisch innerhalb des in der Gasmesszelle 4 enthaltenen Ozons als Vergleichsmaß bestimmt. In einer zweiten Messung wird nach dem Initiieren, d.h. nach dem Einschalten der Quelle 13 für die Aktivierungsstrahlung 14, die dann von dem Ozon generierte Schallwelle bzw. deren Amplitude erfasst und daraus ein Maß für die Konzentration des Ozons abgeleitet. Aus dem Vergleichsmaß und dem Maß wird dann wie zuvor ein Vergleichsmaß für die Konzentration des Sauerstoffs gebildet.

In einer alternativen Ausführungsform wird der Sensor 1 aus Figur 2 zum sehr empfindlichen Nachweis von Stickstoffdioxid verwendet. Dabei bildet das Stickstoffdioxid das erste Fluid im Sinne der vorliegenden Anmeldung. Das Stickstoffdioxid durch eine chemische Reaktion in Stickstoffmonoxid als zweitem Fluid im Sinne der vorliegenden Anmeldung umgewandelt. Durch die chemische Reaktion wird selektiv die Konzentration des Stickstoffdioxids im Innenraum 7 der Gasmesszelle 4 reduziert. Aus einem Vergleich mit und ohne chemische Reaktion kann das Stickstoffdioxid als Ursprung der Schallwelle eindeutig identifiziert werden. Dabei liegt die Anregungsstrahlung im grünen Spektralbereich, während die Aktivierungsstrahlung im ultravioletten Spektralbereich liegt.

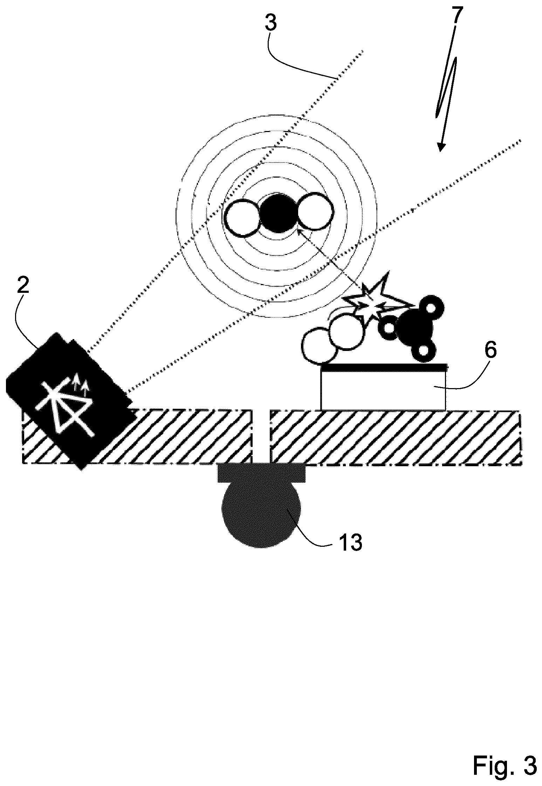

Die Ausführungsform der Figur 3 zeigt eine Variante der Ausführungsform aus Figur 1, wobei in Figur 3 die Gasmesszelle 4 nicht vollständig dargestellt ist. Statt einem Eintrittsfenster 9 ist die Quelle 2 für die Anregungsstrahlung 3 direkt in der Zelle angeordnet, sodass die Anregungsstrahlung 3 nicht erst in die Zelle eingestrahlt werden muss. Ferner weist die Ausführungsform aus Figur 3 auch ein Mikrofon 13 als Schallmesseinrichtung auf.

Die Ausführungsform aus Figur 3 wird zum Erfassen einer Konzentration von Erdgas dargestellt durch Methan in einem in den Innenraum 7 der Gasmesszelle 4 eingeleiteten Gasgemisch verwendet. Dabei bildet das Methan das zweite Fluid im Sinne der vorliegenden Anmeldung. Durch das Heizelement 6 wird eine chemische Reaktion zum Umwandeln des Methans zusammen mit Sauerstoff in Kohlenstoffdioxid initiiert. Das Kohlenstoffdioxid absorbiert die Anregungsstrahlung 3 bei einer Anregungswellenlänge von 4,2 µm.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschrieben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus und der unbestimmte Artikel "eine" oder "ein" schließt eine Mehrzahl nicht aus. Bezugszeichen in den Ansprüchen sind nicht als Beschränkung des Schutzbereichs gedacht.

### Bezugszeichenliste

- 1: Photoakustischer Sensor
- 2: Quelle für die Anregungsstrahlung
- 3: Anregungsstrahlung
- 4: Gasmesszelle
- 5: Gasflusssensor
- 6: Heizelement
- 7: Innenraum
- 8: Seitenwände
- 9: Eintrittsfenster
- 10: Austrittsfenster
- 11: Steuer- und Auswerteeinrichtung
- 12: Mikrofon
- 13: Quelle für die Aktivierungsstrahlung
- 14: Aktivierungsstrahlung
- 15: Linse

## Patentansprüche

1. Verfahren zum Bestimmen einer Eigenschaft eines Fluids mit den Schritten
A) Erzeugen einer mit einer Modulationsfrequenz modulierten elektromagnetischen Anregungsstrahlung (3), wobei eine Trägerfrequenz der Anregungsstrahlung (3) derart gewählt wird, dass ein erstes Fluid die Anregungsstrahlung (3) absorbiert,
B) Beleuchten des ersten Fluids mit der Anregungsstrahlung (3) und
C) Erfassen einer durch eine Absorption der Anregungsstrahlung (3) in dem ersten Fluid erzeugten Schallwelle mit einer Schallmesseinrichtung (5, 12),
D) zeitlich zumindest vor oder während des Beleuchtens des ersten Fluids in Schritt B) Initiieren einer chemischen Reaktion,
E) Umwandeln des ersten Fluids als Reaktionsedukt in ein zweites Fluid als Reaktionsprodukt in der chemischen Reaktion und
F) Bestimmen eines Maßes für eine Eigenschaft des ersten Fluids aus zumindest einer Amplitude oder einer Phase der durch die Absorption der Anregungsstrahlung (3) durch das erste Fluid erzeugten Schallwelle,
**dadurch gekennzeichnet, dass** zeitlich vor dem Initiieren der chemischen Reaktion in Schritt D) die folgenden Schritte G) bis I) ausgeführt werden:
G) Beleuchten des ersten Fluids mit der Anregungsstrahlung (3),
H) Erfassen einer durch die Absorption der Anregungsstrahlung (3) in dem ersten Fluid erzeugten Schallwelle mit der Schallmesseinrichtung (5, 12),
I) Bestimmen eines Vergleichsmaßes für die Eigenschaft des ersten Fluids aus zumindest der Amplitude oder der Phase der durch die Absorption der Anregungsstrahlung (3) durch das erste Fluid erzeugten Schallwelle und
dass das Verfahren weiterhin den Schritt umfasst
J) Bestimmen eines differenziellen Maßes für die Eigenschaft des ersten Fluids aus dem Maß für die Eigenschaft des ersten Fluids aus Schritt F) und dem Vergleichsmaß für die Eigenschaft des ersten Fluids aus Schritt I).

2. Verfahren nach dem vorhergehenden Anspruch, wobei das Initiieren der chemischen Reaktion in Schritt D) durch zumindest einen der folgenden Schritte erfolgt
- Zuführen von thermischer Energie in das Reaktionsedukt,
- Bestrahlen des Reaktionsedukts mit elektromagnetischer Aktivierungsstrahlung,
- Einbringen eines Katalysators in das Reaktionsedukt,
- Aktivieren eines Katalysators in dem Reaktionsedukt und
- chemisches Aktivieren des Reaktionsedukts.

3. Verfahren nach einem der vorhergehenden Ansprüche, wobei das Initiieren der chemischen Reaktion in Schritt D) durch Bestrahlen des Reaktionsedukts mit der elektromagnetischen Anregungsstrahlung (3) erfolgt, wobei die Anregungsstrahlung (3) eine zwischen einem ersten Wert zeitlich vor dem Schritt D) und einem zweiten Wert zeitlich während einer Ausführung des Schritts D) veränderbare Modulationstiefe aufweist.

4. Verfahren nach einem der vorhergehenden Ansprüche, wobei die Eigenschaft des ersten Fluids ein Vorhandensein oder eine Konzentration des ersten Fluids ist oder wobei die Eigenschaft des zweiten Fluids ein Vorhandensein oder eine Konzentration des zweiten Fluids ist.

5. Verwendung des Verfahrens nach einem der vorhergehenden Ansprüche zum Nachweis von Stickstoffdioxid oder zum Erfassen einer Konzentration von Stickstoffdioxid, wobei das erste Fluid Stickstoffdioxid ist und das zweite Fluid Stickstoffmonoxid ist, wobei das Stickstoffdioxid in Schritt F) durch eine chemische Reaktion in das Stickstoffmonoxid umgewandelt wird, wobei die chemische Reaktion vorzugsweise durch Bestrahlen mit elektromagnetischer Aktivierungsstrahlung initiiert wird.

6. Vorrichtung (1) zum Bestimmen einer Eigenschaft eines Fluids mit
dem ersten Fluid,
einer Quelle (2), die derart eingerichtet ist, dass die Quelle (2) in einem Betrieb der Vorrichtung (1) mit einer Modulationsfrequenz modulierte elektromagnetische Anregungsstrahlung (3) erzeugt, wobei eine Trägerfrequenz der Anregungsstrahlung (3) derart gewählt ist, dass das erstes Fluid die Anregungsstrahlung (3) absorbiert,
wobei die Quelle (2) derart angeordnet ist, dass in dem Betrieb der Vorrichtung (1) das erste Fluid mit der Anregungsstrahlung beleuchtet wird, und
einer Schallmesseinrichtung (5, 12), wobei die Schallmesseinrichtung derart eingerichtet und angeordnet ist, dass in dem Betrieb der Vorrichtung (1) eine durch eine Absorption der Anregungsstrahlung (3) in dem ersten Fluid erzeugte Schallwelle mit der Schallmesseinrichtung (5, 12) erfasst wird,
einer Aktivierungseinrichtung (6, 13) und
einer Steuer- und Auswerteeinrichtung (11),
wobei die Steuer- und Auswerteeinrichtung (11) derart wirksam mit der Schallmesseinrichtung (5, 12) verbunden ist, dass die Steuer- und Auswerteeinrichtung (11) in dem Betrieb der Vorrichtung (1) ein Messsignal von der Schallmesseinrichtung (5, 12) erhält,
wobei die Steuer- und Auswerteeinrichtung (11) derart wirksam mit der Aktivierungseinrichtung verbunden ist, dass die Aktivierungseinrichtung (5, 12) in dem Betrieb der Vorrichtung (1) von der Steuer- und Auswerteeinrichtung (11) steuerbar ist, und
wobei
die Aktivierungseinrichtung (6, 13) derart eingerichtet und angeordnet ist, dass die Aktivierungseinrichtung (6, 13) in dem Betrieb der Vorrichtung (1) eine chemische Reaktion zum Umwandeln des ersten Fluids als Reaktionsedukt in ein zweites Fluid als Reaktionsprodukt initiiert, und die Steuer- und Auswerteeinrichtung (11) derart eingerichtet ist, dass die Steuer- und Auswerteeinrichtung (11) in dem Betrieb der Vorrichtung (1) aus dem Messsignal der durch die Absorption der Anregungsstrahlung (3) durch das erste Fluid erzeugten Schallwelle ein Maß für eine Eigenschaft des ersten Fluids bestimmt,
**dadurch gekennzeichnet, dass**
die Steuer- und Auswerteeinrichtung (11) derart eingerichtet ist, dass die Steuer- und Auswerteeinrichtung (11) in dem Betrieb der Vorrichtung (1) zeitlich vor dem Initiieren der chemischen Reaktion aus dem Messsignal der durch die Absorption der Anregungsstrahlung (3) durch das erste Fluid erzeugten Schallwelle ein Vergleichsmaß für die Eigenschaft des ersten Fluids bestimmt, und ein differenzielles Maß für die Eigenschaft des ersten Fluids aus dem Maß für die Eigenschaft des ersten Fluids und dem Vergleichsmaß für die Eigenschaft des ersten Fluids bestimmt.

7. Vorrichtung (1) nach dem vorhergehenden Anspruch, wobei die Aktivierungseinrichtung (6, 13) ausgewählt ist aus
- einer Heizeinrichtung (6) zum Zuführen von thermischer Energie in das Reaktionsedukt,
- einer Quelle (13) für elektromagnetische Aktivierungsstrahlung (14) zum Bestrahlen des Reaktionsedukts,
- einem aktivierbaren Katalysator (6),
- einer Einrichtung zum Einspritzen einer chemischen Substanz zum Aktivieren des Reaktionsedukts
oder einer Kombination davon.

8. Vorrichtung nach Anspruch 6 oder 7, wobei die Vorrichtung eine Gasmesszelle (4) aufweist, wobei die Gasmesszelle (4) derart ausgestaltet ist, dass in der Gasmesszelle (4) das Reaktionsedukt und das Reaktionsprodukt aufnehmbar ist und die chemische Reaktion in der Gasmesszelle (4) ablaufen kann, und wobei die Schallmesseinrichtung (5, 12) in der Gasmesszelle angeordnet ist.

9. Vorrichtung (1) nach dem vorhergehenden Anspruch, wobei die Gasmesszelle (4) einen akustischen Resonator mit einer Eigenfrequenz bildet, wobei die Eigenfrequenz gleich der Modulationsfrequenz ist.

## Claims

1. A method for determining a property of a fluid comprising the steps:
A) generating an electromagnetic excitation radiation (3) modulated with a modulation frequency, wherein a carrier frequency of the excitation radiation (3) is selected such that a first fluid absorbs the excitation radiation (3),
B) illuminating the first fluid with the excitation radiation (3) and
C) detecting an acoustic wave generated by an absorption of the excitation radiation (3) in the first fluid with a sound measuring device (5, 12),
D) in time, at least before or during the illuminating of the first fluid in step B) initiating a chemical reaction,
E) converting the first fluid as reactant into a second fluid as reaction product in the chemical reaction and
F) determining a measure for a property of the first fluid from at least an amplitude or a phase of the acoustic wave generated by the absorption of the excitation radiation (3) by the first fluid,
**characterized in that**, in time, the following steps G) to I) are carried out before the initiating of the chemical reaction in step D):
G) illuminating the first fluid with the excitation radiation (3),
H) detecting an acoustic wave generated by the absorption of the excitation radiation (3) in the first fluid with the sound measuring device (5, 12),
I) determining a comparison measure for the property of the first fluid from at least the amplitude or the phase of the acoustic wave generated by the absorption of the excitation radiation (3) by the first fluid and
that the method further comprises the step:
J) determining a differential measure for the property of the first fluid from the measure for the property of the first fluid from step F) and the comparison measure for the property of the first fluid from step I).

2. The method according to the preceding claim, wherein the initiating of the chemical reaction in step D) is effected by at least one of the following steps:
- supplying thermal energy into the reactant,
- irradiating the reactant with electromagnetic activation radiation,
- introducing a catalyst into the reactant,
- activating a catalyst in the reactant and
- chemical activating of the reactant.

3. The method according to one of the preceding claims, wherein the initiating of the chemical reaction in step D) is effected by irradiating the reactant with the electromagnetic excitation radiation (3), wherein the excitation radiation (3) has a modulation depth variable between a first value temporally before step D) and a second value temporally during an execution of step D).

4. The method according to one of the preceding claims, wherein the property of the first fluid is a presence or a concentration of the first fluid or wherein the property of the second fluid is a presence or a concentration of the second fluid.

5. Use of the method according to one of the preceding claims for detecting nitrogen dioxide or for detecting a concentration of nitrogen dioxide, wherein the first fluid is nitrogen dioxide and the second fluid is nitrogen monoxide, wherein the nitrogen dioxide in step E) is converted by a chemical reaction into the nitrogen monoxide, wherein the chemical reaction is preferably initiated by irradiation with electromagnetic activation radiation.

6. A device (1) for determining a property of a fluid with
the first fluid,
a source (2) which is configured such that the source (2) in an operation of the device (1) generates electromagnetic excitation radiation (3) modulated with a modulation frequency, wherein a carrier frequency of the excitation radiation (3) is selected such that the first fluid absorbs the excitation radiation (3),
wherein the source (2) is arranged such that in the operation of the device (1) the first fluid is illuminated with the excitation radiation, and
a sound measuring device (5, 12), wherein the sound measuring device is configured and arranged such that in the operation of the device (1) an acoustic wave generated by an absorption of the excitation radiation (3) in the first fluid is detected with the sound measuring device (5, 12),
an activation device (6, 13) and
a control and evaluation device (11),
wherein the control and evaluation device (11) is operatively connected to the sound measuring device (5, 12) such that the control and evaluation device (11) in the operation of the device (1) receives a measurement signal from the sound measuring device (5, 12),
wherein the control and evaluation device (11) is operatively connected to the activation device such that the activation device (5, 12) in the operation of the device (1) is controllable by the control and evaluation device (11), and
wherein
the activation device (6, 13) is configured and arranged such that the activation device (6, 13) in the operation of the device (1) initiates a chemical reaction for converting the first fluid as reactant into a second fluid as reaction product, and
the control and evaluation device (11) is configured such that the control and evaluation device (11) in the operation of the device (1) determines from the measurement signal of the acoustic wave generated by the absorption of the excitation radiation (3) by the first fluid a measure for a property of the first fluid,
**characterized in that**
the control and evaluation device (11) is configured such that the control and evaluation device (11) in the operation of the device (1) temporally before the initiating of the chemical reaction determines a comparison measure for the property of the first fluid from the measurement signal of the acoustic wave generated by the absorption of the excitation radiation (3) by the first fluid and determines a differential measure for the property of the first fluid from the measure for the property of the first fluid and the comparison measure for the property of the first fluid.

7. The device (1) according to the preceding claim, wherein the activation device (6, 13) is selected from
- a heating device (6) for supplying thermal energy into the reactant,
- a source (13) for electromagnetic activation radiation (14) for irradiating the reactant,
- an activatable catalyst (6),
- a device for injecting a chemical substance for activating the reactant or a combination thereof.

8. The device according to claim 6 or 7, wherein the device comprises a gas measurement cell (4), wherein the gas measurement cell (4) is configured such that in the gas measurement cell (4) the reactant and the reaction product are receivable and the chemical reaction can take place in the gas measurement cell (4), and wherein the sound measuring device (5, 12) is arranged in the gas measurement cell.

9. The device (1) according to the preceding claim, wherein the gas measurement cell (4) forms an acoustic resonator with a natural frequency, wherein the natural frequency is equal to the modulation frequency.

## Revendications

1. Procédé pour déterminer une propriété d'un fluide avec les étapes
A) produire un rayonnement d'excitation électromagnétique (3) modulé avec une fréquence de modulation, une fréquence porteuse du rayonnement d'excitation (3) étant choisi de façon qu'un premier fluide absorbe le rayonnement d'excitation (3),
B) éclairer le premier fluide avec le rayonnement d'excitation (3) et
C) capter une onde acoustique engendrée dans le premier fluide par une absorption du rayonnement d'excitation (3), moyennant un dispositif de mesure acoustique (5, 12),
D) au moins avant ou pendant l'éclairage du premier fluide à l'étape B), initier une réaction chimique,
E) modifier le premier fluide en tant que produit de départ de réaction en un deuxième fluide en tant que produit résultant de réaction dans la réaction chimique et
F) déterminer une mesure pour une propriété du premier fluide à partir d'au moins une amplitude ou une phase de l'onde acoustique engendrée par l'absorption du rayonnement d'excitation (3) par le premier fluide,
**caractérisé en ce que**, avant l'initiation de la réaction chimique à l'étape D), les étapes suivantes G) à I) sont mises en oeuvre :
G) éclairer le premier fluide avec le rayonnement d'excitation (3),
H) capter une onde acoustique engendrée dans le premier fluide par l'absorption du rayonnement d'excitation (3) avec le dispositif de mesure acoustique (5, 12),
I) déterminer une mesure comparative pour la propriété du premier fluide à partir d'au moins l'amplitude ou la phase de l'onde acoustique engendrée par l'absorption du rayonnement d'excitation (3) par le premier fluide et
**en ce que** le procédé comprend en outre l'étape
J) déterminer une mesure différentielle pour la propriété du premier fluide à partir de la mesure pour la propriété du premier fluide de l'étape F) et la mesure comparative pour la propriété du premier fluide de l'étape I).

2. Procédé selon la revendication précédente, l'initiation de la réaction chimique à l'étape D) étant effectuée par au moins une des étapes suivantes
- apporter de l'énergie thermique au produit de départ de réaction,
- irradier le produit de départ de réaction avec un rayonnement électromagnétique d'activation,
- introduire un catalyseur dans le produit de réaction,
- activer un catalyseur dans le produit de départ de réaction et
- activer chimiquement le produit de départ de réaction.

3. Procédé selon l'une des revendications précédentes, l'initiation de la réaction chimique à l'étape D) étant effectuée par irradiation du produit de départ de réaction avec le rayonnement d'excitation (3), le rayonnement d'excitation (3) ayant une profondeur de modulation variable entre une première valeur avant l'étape D) et une deuxième valeur pendant une mise en œuvre de l'étape D).

4. Procédé selon l'une des revendications précédentes, la propriété du premier fluide étant une présence ou une concentration du premier fluide et la propriété du deuxième fluide étant une présence ou une concentration du deuxième fluide.

5. Utilisation du procédé selon l'une des revendications précédentes pour la détection de dioxyde d'azote ou pour capter une concentration de dioxyde d'azote, le premier fluide étant du dioxyde d'azote et le deuxième fluide étant du monoxyde d'azote, le dioxyde d'azote étant transformé à l'étape F) par une réaction chimique en monoxyde d'azote, la réaction chimique étant initiée de préférence par une irradiation avec un rayonnement d'activation électromagnétique.

6. Dispositif (1) pour déterminer une propriété d'un fluide avec
le premier fluide,
une source (2) qui est agencée de façon telle que la source (2) engendre, lors d'un fonctionnement du dispositif (1), un rayonnement d'excitation électromagnétique (3) modulé avec une fréquence de modulation, une fréquence porteuse du rayonnement étant choisie de façon que le premier fluide absorbe le rayonnement d'excitation (3),
la source (2) étant disposée de façon que, lors du fonctionnement du dispositif (1), le premier fluide soit éclairé par le rayonnement d'excitation, et
un dispositif de mesure acoustique (5, 12), le dispositif de mesure acoustique étant agencé et disposé de façon que, lors du fonctionnement du dispositif (1), une onde acoustique engendrée dans le premier fluide par une absorption du rayonnement d'excitation (3) soit captée par le dispositif de mesure acoustique (5, 12),
un dispositif d'activation (6, 13) et
un dispositif de commande et d'exploitation (11),
le dispositif de commande et d'exploitation (11) étant relié effectivement au dispositif de mesure acoustique (5, 12) de façon telle que, lors du fonctionnement du dispositif (1), le dispositif de commande et d'exploitation (11) reçoive un signal de mesure provenant du dispositif de mesure acoustique (5, 12),
le dispositif de commande et d'exploitation (11) étant relié effectivement au dispositif d'activation de façon telle que, lors du fonctionnement du dispositif (1), le dispositif d'activation (5, 12) puisse être commandé par le dispositif de commande et d'exploitation (11), et
le dispositif d'activation (6, 13) étant agencé et disposé de façon que, lors du fonctionnement du dispositif (1), le dispositif d'activation (6, 13) initie une réaction chimique pour la transformation du premier fluide en tant que produit de départ de réaction en un deuxième fluide comme produit résultant de réaction, et
le dispositif de commande et d'exploitation (11) étant agencé de façon que, lors du fonctionnement du dispositif (1), le dispositif de commande et d'exploitation (11) détermine à partir du signal de mesure de l'onde acoustique engendrée par l'absorption du rayonnement d'excitation (3) par le premier fluide, une mesure pour la propriété du premier fluide,
**caractérisé en ce que**
le dispositif de commande et d'exploitation (11) est agencé de façon que, lors du fonctionnement du dispositif (1), le dispositif de commande et d'exploitation (11) détermine avant l'initiation de la réaction chimique, à partir du signal de mesure de l'onde acoustique engendrée par l'absorption du rayonnement d'excitation (3) par le premier fluide, une mesure de comparaison pour la propriété du premier fluide et détermine une mesure différentielle pour la propriété du premier fluide à par de la mesure pour la propriété du premier fluide et de la mesure de comparaison pour la propriété du premier fluide.

7. Dispositif (1) selon la revendication précédente, le dispositif d'activation (6, 13) étant choisi parmi
- un dispositif de chauffage (6) à apporter de l'énergie thermique au produit de départ de réaction,
- une source (13) de rayonnement d'activation électromagnétique (14) pour irradier le produit de départ de réaction,
- un catalyseur (6) pouvant être activé,
- un dispositif pour injecter une substance chimique pour activer le produit de départ de réaction
ou une combinaison de ceux-ci.

8. Dispositif selon la revendication 6 ou 7, le dispositif comprenant une cellule de mesure de gaz (4), la cellule de mesure de gaz (4) étant agencé de façon que le produit de départ de réaction et le produit résultant de réaction puissent être reçus dans la cellule de mesure de gaz (4), le dispositif de mesure acoustique (5, 12) étant disposé dans la cellule de mesure de gaz.

9. Dispositif (1) selon la revendication précédente, la cellule de mesure de gaz (4) formant un résonateur acoustique avec une fréquence propre, la fréquence propre étant égale à la fréquence de modulation.
